# EUROPÄISCHE PATENTSCHRIFT

(11) **EP 1 496 890 B1**
(45) Veröffentlichungstag und Bekanntmachung des Hinweises auf die Patenterteilung: **02.09.2009**
(21) Anmeldenummer: 02796583.9
(22) Anmeldetag: 09.12.2002
(51) Int. Cl.: A61K 31/40, A61K 31/445, A61K 31/5375, A61K 31/55, C07D 295/092, A61P 25/04, A61P 1/08, A61P 25/28, A61P 9/00

(54) **SUBSTITUIERTE BETA-AMINOALKOHOLE ALS ARZNEIMITTEL**
SUBSTITUTED BETA-AMINOALCOHOLS USED AS MEDICAMENTS
BETA-AMINOALCOOLS SUBSTITUES UTILISES EN TANT QUE MEDICAMENTS

(30) Priorität: 14.12.2001 DE 10164581
(43) Veröffentlichungstag der Anmeldung: 19.01.2005
(73) Patentinhaber: Grünenthal GmbH, 52078 Aachen (DE)
(72) Erfinder: URAGG, Heinz, 52223 Stolberg (DE); MAUL, Corinna, 52066 Aachen (DE); BUSCHMANN, Helmut, E-08950 Esplugues de Llobregat (ES); SUNDERMANN, Bernd, 52066 Aachen (DE); HENNIES, Hagen-Heinrich, 52152 Simmerath (DE)
(74) Vertreter: Kutzenberger, Helga
(86) Internationale Anmeldenummer: PCT/EP2002/013910
(87) Internationale Veröffentlichungsnummer: WO 2003/051353

(56) Entgegenhaltungen:
- DE-B- 1 146 068
- DATABASE CROSSFIRE BEILSTEIN [Online] Beilstein Institut zur Förderung der Chemischen Wissenschaften, Frankfurt am Main, DE; TAKAHASHI, HORI: Database accession no. 117263 & 117658 XP002232916 & YAKUGAKU ZASSHI, Bd. 74, 1954, Seite 1141
- DATABASE CROSSFIRE BEILSTEIN [Online] Beilstein Institut zur Förderung der Chemischen Wissenschaften, Frankfurt am Main, DE; NOBLES, THOMPSON: Database accession no. 1317089 XP002232917 & J. PHARM. SCI., Bd. 53, 1964, Seite 1554
- PATENT ABSTRACTS OF JAPAN vol. 013, no. 379, 22. August 1989 (1989-08-22) & JP 01 131170 A (NIPPON KAYAKU CO. LTD.), 24. Mai 1989 (1989-05-24)
- CHEMICAL ABSTRACTS, vol. 80, no. 3, 21. Januar 1974 (1974-01-21) Columbus, Ohio, US; abstract no. 14610n, E. RAVINA ET AL.: "4,4-Disubstituted cyclohexanes. VI. Tertiary aminomethyl cyclohexanols as antispasmodics" Seite 404; XP002232907 & AN. QUIM., Bd. 69, Nr. 7-8, 1973, Seiten 915-920,
- CHEMICAL ABSTRACTS, vol. 128, no. 9, 2. März 1998 (1998-03-02) Columbus, Ohio, US; abstract no. 102001e, P. HORSTMANN, B. UNTERHALT: "Amino alcohols related to tramadol (tramal)" Seite 572; XP002232908 & ARCH. PHARM. (WEINHEIM, GER.), Bd. 330, Nr. 11, 1997, Seiten 362-364,
- CHEMICAL ABSTRACTS, vol. 108, no. 9, 29. Februar 1988 (1988-02-29) Columbus, Ohio, US; abstract no. 75179x, F. EIDEN, M. SCHMIDT: "4-Pyrans. 122. CNS-active phenylpyrans: 3-(dimethylaminomethyl)-4-(3-methoxyphenyl )tetrahydro-4-pyranol and 4-thiopyranol" Seite 660; XP002232909 & ARCH. PHARM. (WEINHEIM, GER.), Bd. 320, Nr. 11, 1987, Seiten 1099-1103,
- CHEMICAL ABSTRACTS, vol. 106, no. 15, 13. April 1987 (1987-04-13) Columbus, Ohio, US; abstract no. 119635h, O. CAAMANO ET AL.: "Synthesis of semi-rigid models of butyrophenone neuroleptic drugs. I. Evaluation of a route leading to phenyl trans-2-(1-piperidinylmethyl)cyclohexyl ketone" Seite 621; XP002232910 & AN. QUIM., SER. C, Bd. 81, Nr. 3, 1985, Seiten 271-273,
- CHEMICAL ABSTRACTS, vol. 77, no. 11, 11. September 1972 (1972-09-11) Columbus, Ohio, US; abstract no. 74977k, T. KAMETANI ET AL.: "Syntheses of analgesics. XXVIII. Syntheses of 4-Amino-3-methyl-1,2-diphenyl-2-propionylo xybutane derivatives" Seite 459; XP002232911 & YAKUGAKU ZASSHI, Bd. 92, Nr. 4, 1972, Seiten 421-430,
- CHEMICAL ABSTRACTS, vol. 73, no. 11, 14. September 1970 (1970-09-14) Columbus, Ohio, US; abstract no. 55772r, P. PARIMOO, NOBLES, W. L.: "New compounds: some potential chemotherapeutic agents derived from aralkyl ketones" Seite 297; XP002232912 & J. PHARM. SCI., Bd. 59, Nr. 7, 1970, Seiten 1038-1041,
- CHEMICAL ABSTRACTS, vol. 67, no. 23, 4. Dezember 1967 (1967-12-04) Columbus, Ohio, US; abstract no. 108504q, K. H. BOLTZE, H. D. DELL: "Substituted amino alcohols from Grignard reactions of aminomethylated 3,3,5-trimethylcyclohexanone and 3,3,5-trimethyl cyclohexyl methyl ketone and their pharmacological effects. I. Methyl-substituted cyclohexane derivatives" Seite 10225; XP002232913 & ARZNEIM.-FORSCH., Bd. 17, Nr. 6, 1967, Seiten 731-735,
- CHEMICAL ABSTRACTS, vol. 66, no. 9, 27. Februar 1967 (1967-02-27) Columbus, Ohio, US; abstract no. 37734f, C. CASAGRANDE, G. FERRARI: "Esters of 1-amino-2,3-diphenyl-3-pentanol and -heptanol" Seite 3589; XP002232914 & FARMACO ED. SCI., Bd. 21, Nr. 10, 1966, Seiten 713-722,
- CHEMICAL ABSTRACTS, vol. 60, no. 5, 2. März 1964 (1964-03-02) Columbus, Ohio, US; B. REICHERT, A. MAYR: "Aminomethylation products of alkylcyclohexanones and their reactions" Spalte 5356f; XP002232915 & ARZNEIMITTEL-FORSCH., Bd. 13, 1963, Seiten 991-999,

## Beschreibung

Die vorliegende Erfindung betrifft Arzneimittel enthaltend substituierte Aminoalkohole und die Verwendung von substituierten Aminoalkoholen zur Herstellung von Arzneimitteln sowie substituierte 2-(Aminomethyl)cycloalkan-1-ol-Verbindungen und Verfahren zu deren Herstellung.

Die Behandlung von Schmerz hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Aufgabe der Erfindung war es daher, neue Arzneimittel zur Behandlung von Schmerz zur Verfügung zu stellen.

Erfindungsgemäß wird diese Aufgabe durch Bereitstellung der Arzneimittel enthaltend Aminoalkohole der nachstehend angegebenen Formel I gelöst, da diese Arzneimittel insbesondere eine ausgeprägte analgetische Wirkung aufweisen und zur Behandlung von Schmerz, als Antiemetikum und/oder Nootropikum (Neurotropikum), zur Behandlung von cardiovaskulären Erkrankungen, Haminkontinenz, Diarrhöe, Pruritus, Alkohol- und/oder Drogen- und/oder Medikamentenabhängigkeit und/oder Entzündungen, zur Behandlung von Depressionen, zur Vigilanzsteigerung und/oder Libidosteigerung eingesetzt werden können.

Gegenstand der Erfindung sind daher Arzneimittel enthaltend Aminoalkohole der allgemeinen Formel I, 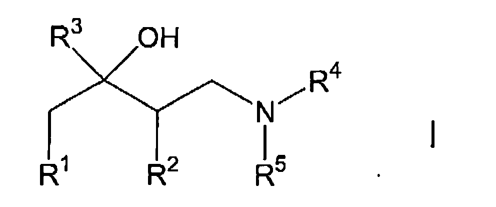 worin
R¹ und R², gleich oder verschieden, für jeweils einen linearen oder verzweigten, gesättigten oder ungesättigten aliphatischen C₁₋₃-Rest stehen oder zusammen eine (CH₂)ₙ-Kette bilden, wobei n für eine ganze Zahl von 2-9 steht,

R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest ausgewählt aus der Gruppe umfassend Pyrrolyl-, Furyl- (Furanyl-), Thienyl- (Thiophenyl-), Pyrazolyl-, Imidazolyl-, Thiazolyl-, Oxazolyl-, Isoxazolyl-, Pyridinyl-, Pyridazinyl-, Pyrimidinyl-, Pyrazinyl- und Pyranyl steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert und/oder über eine lineare oder verzweigte, gesättigte oder ungesättigte aliphatische C₁₋₃-Brücke gebunden sein kann,

R⁴ und R⁵ zusammen eine (CH₂)ₘ-Kette bilden, wobei m für 6 steht und gegebenenfalls eine oder mehrere CH₂-Gruppen durch Sauerstoff ersetzt sein können,

in Form ihrer Diastereomere, ihrer Enantiomere und Gemische davon - einschließlich ihrer Racemate - sowie in Form entsprechender Basen, Salze und Solvate als Wirkstoff und gegebenenfalls physiologisch verträgliche Hilfsstoffe.

Bevorzugt sind Arzneimittel enthaltend wenigstens eine Verbindung der allgemeinen Formel I, worin
R¹ und R² jeweils für eine Methylgruppe stehen oder zusammen eine (CH₂)ₙ-Kette bilden, wobei n für 2, 3, 4, 5 oder 9 steht,

R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann,

R⁴ und R⁵ zusammen eine (CH₂)ₘ-Kette bilden, wobei m für 6 steht.

Besonders bevorzugt sind Arzneimittel enthaltend wenigstens eine Verbindung der allgemeinen Formel II, 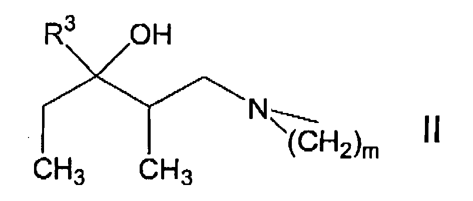 worin
m für 6 steht.

Besonders bevorzugt sind Arzneimittel enthaltend wenigstens eine Verbindung der allgemeinen Formel III, 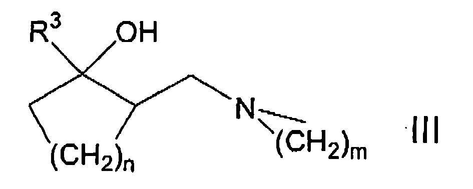 worin
n für eine ganze Zahl von 2-9 und m für 6 stehen. Weiter bevorzugt sind Arzneimittel enthaltend wenigstens eine Verbindung der allgemeinen Formel II oder III, worin

R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest ausgewählt aus der Gruppe umfassend Pyrrolyl-, Furyl- (Furanyl-), Thienyl- (Thiophenyl-), Pyrazolyl-, Imidazolyl-, Thiazolyl-, Oxazolyl-, Isoxazolyl-, Pyridinyl-, Pyridazinyl-, Pyrimidinyl-, Pyrazinyl- und Pyranyl steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert und/oder über eine lineare oder verzweigte, gesättigte oder ungesättigte aliphatische C₁₋₃-Brücke gebunden sein kann
und die übrigen Symbole die vorstehend genannte Bedeutung haben.

Weiter bevorzugt sind Arzneimittel enthaltend wenigstens eine Verbindung der allgemeinen Formel II oder III, worin

R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann
und die übrigen Symbole die vorstehend genannte Bedeutung haben.

Unter einem Heteroarylrest wird ein gegebenenfalls ein- oder mehrfach substituierter Heteroarylrest ausgewählt aus der Gruppe umfassend Pyrrolyl-, Furyl- (Furanyl-), Thienyl- (Thiophenyl-), Pyrazolyl-, Imidazolyl-, Thiazolyl-, Oxazolyl-, Isoxazoyl-, Pydridinyl-, Pyridazinyl-, Pyrimidinyl-, Pyrazinyl- und Pyranyl verstanden. Die Bindung kann über jedes beliebige, bindungsfähige Ringatom erfolgen. Die gegebenenfalls vorhandenen Substituenten können gleich oder verschieden sein und an jedes beliebige, bindungsfähige Ringatom gebunden sein.

Unter einem Arylrest wird ein gegebenenfalls ein- oder mehrfach substituierte Phenylrest verstanden. Die Bindung kann über jedes beliebige, bindungsfähige Ringatom erfolgen. Die gegebenenfalls vorhandenen Substituenten können gleich oder verschieden sein und an jedes beliebige, bindungsfähige Ringatom gebunden sein.

Ganz besonders bevorzugt sind Arzneimittel enthaltend wenigstens eine Verbindung ausgewählt aus der Gruppe umfassend
2-Azepan-1-ylmethyl-1-phenyl-cyclohexanol
2-Azepan-1-ylmethyl-1-benzyl-cyclohexanol
2-Azepan-1-ylmethyl-1-(4-fluor-3-methyl-phenyl)-cyclohexanol
2-Azepan-1-ylmethyl-1-o-tolyl-cyclohexanol
2-Azepan-1-ylmethyl-1-vinyl-cyclohexanol
2-Azepan-1-ylmethyl-1-(4-tert-butyl-phenyl)-cyclohexanol
2-Azepan-1-ylmethyl-1-cyclopentyl-cyclohexanol
2-Azepan-1-ylmethyl-1-m-tolyl-cyclohexanol
2-Azepan-1-ylmethyl-bicyclohexyl-1-ol
2-Azepan-1-ylmethyl-1-(4-fluor-phenyl)-cyclohexanol
2-Azepan-1-ylmethyl-1-phenethyl-cyclohexanol
2-Azepan-1-ylmethyl-1-thiophen-2-yl-cyclohexanol
2-Azepan-1-ylmethyl-1-(2,4-dichlor-phenyl)-cyclohexanol
2-Azepan-1-ylmethyl-1-(3-methoxy-phenyl)-cyclohexanol
2-Azepan-1-ylmethyl-1-(3-phenyl-propyl)-cyclohexanol
2-Azepan-1-ylmethyl-1-p-tolyl-cyclohexanol
2-Azepan-1-ylmethyl-1-(4-methoxy-phenyl)-cyclohexanot
2-Azepan-1-ylmethyl-1-phenyl-cyclopentanol
2-Azepan-1-ylmethyl-1-(4-chlor-phenyl)-cyclopentanol
2-Azepan-1-ylmethyl-1-benzyl-cyclopentanol
2-Azepan-1-ylmethyl-1-(4-fluor-3-methyl-phenyl)-cyclopentanol
2-Azepan-1-ylmethyl-1-o-tolyl-cyclopentanol
2-Azepan-1-ylmethyl-1-vinyl-cyclopentanol
2-Azepan-1-ylmethyl-1-(4-tert-butyl-phenyl)-cyclopentanol
2-Azepan-1-ylmethyl-bicyclopentyl-1-ol
2-Azepan-1-ylmethyl-1-m-tolyl-cyclopentanol
2-Azepan-1-ylmethyl-1-cyclohexyl-cyclopentanol
2-Azepan-1-ylmethyl-1-(4-fluor-phenyl)-cydopentanol
2-Azepan-1-ylmethyl-1-phenethyl-cyclopentanol
2-Azepan-1-ylmethyl-1-phenylethynyl-cyclopentanol
2-Azepan-1-ylmethyl-1-thiophen-2-yl-cyclopentanol
2-Azepan-1-ylmethyl-1-(3-methoxy-phenyl)-cyclopentanol
2-Azepan-1-ylmethyl-1-(3-phenyl-propyl)-cyclopentanol
2-Azepan-1-ylmethyl-1-p-tolyl-cyclopentanol
2-Azepan-1-ylmethyl-1-(4-methoxy-phenyl)-cyclopentanol
2-Azepan-1-ylmethyl-1-phenyl-cycloheptanol
2-Azepan-1-ylmethyl-1-benzyl-cycloheptanol
2-Azepan-1-ylmethyl-1-(4-fluor-3-methyl-phenyl)-cycloheptanol
2-Azepan-1-ylmethyl-1-o-tolyl-cycloheptanol
2-Azepan-1-ylmethyl-1-vinyl-cycloheptanol
2-Azepan-1-ylmethyl-1-(4-tert-butyl-phenyl)-cycloheptanol
2-Azepan-1-ylmethyl-1-cyclopentyl-cycloheptanol
2-Azepan-1-ylmethyl-1-m-tolyl-cycloheptanol
2-Azepan-1-ylmethyl-1-cyclohexyl-cycloheptanol
2-Azepan-1-ylmethyl-1-(4-fluor-phenyl)-cycloheptanol
2-Azepan-1-ylmethyl-1-phenethyl-cycloheptanol
2-Azepan-1-ylmethyl-1-phenylethynyl-cycloheptanol
2-Azepan-1-ylmethyl-1-thiophen-2-yl-cycloheptanol
2-Azepan-1-ylmethyl-1-(2,4-dichlor-phenyl)-cycloheptanol
2-Azepan-1-ylmethyl-1-(3-methoxy-phenyl)-cycloheptanol
2-Azepan-1-ylmethyl-1-(3-phenyl-propyl)-cycloheptanol
2-Azepan-1-ylmethyl-1-p-tolyl-cycloheptanol
2-Azepan-1-ylmethyl-1-(4-methoxy-phenyl)-cycloheptanol
2-Azepan-1-ylmethyl-1-(3-phenyl-propyl)-cyclopentanol
2-Azepan-1-ylmethyl-1-(2,3-dichlor-phenyl)-cyclopentanol
2-Azepan-1-ylmethyl-1-benzyl-cyclododecanol
2-Azepan-1-ylmethyl-1-vinyl-cyclododecanol
2-Azepan-1-ylmethyl-1-(4-tert-butyl-phenyl)-cyclododecanol
2-Azepan-1-ylmethyl-1-phenethyl-cyclododecanol
2-Azepan-1-ylmethyl-1-(3-phenyl-propyl)-cyclododecanol
2-Azepan-1-ylmethyl-1-(3-fluor-phenyl)-cyclohexanol
2-Azepan-1-ylmethyl-1-(3-chlor-phenyl)-cyclohexanol
2-Azepan-1-ylmethyl-1-cyclohexylmethyl-cyclopentanol
2-Azepan-1-ylmethyl-1-(2-chlor-6-fluor-benzyl)-cyclopentanol
2-Azepan-1-ylmethyl-1-(2,5-dimethyl-benzyl)-cyclopentanol
2-Azepan-1-ylmethyl-1-(4-fluor-benzyl)-cycloheptanol
1-Azepan-1-yl-3-cyclohexylmethyl-2-methyl-pentan-3-ol
1-Azepan-1-yl-3-(5-fluor-2-methoxy-phenyl)-2-methyl-pentan-3-ol
2-Azepan-1-ylmethyl-1-(3-methoxy-benzyl)-cycloheptanol
2-Azepan-1-ylmethyl-1-(3-methoxy-benzyl)-cycloheptanol
2-Azepan-1-ylmethyl-1-(3-fluor-benzyl)-cyclopentanol
2-Azepan-1-ylmethyl-1-(3-phenyl-propyl)-cyclooctanol.

Die erfindungsgemäßen Arzneimittel können die Verbindungen der Formel I, vorzugsweise der allgemeinen Formel II oder III, in Form ihrer Enantiomeren, ihrer Diastereomeren oder in Form eines Gemisches aus wenigstens zwei der vorstehend genannten Stereoisomeren - einschließlich ihrer Racemate - enthalten.

Vorzugsweise eignen sich die erfindungsgemäßen Arzneimittel zur Behandlung von Schmerz, als Antiemetikum und/oder Nootropikum (Neurotropikum), zur Behandlung von cardiovaskulären Erkrankungen, Harninkontinenz, Diarrhöe, Pruritus, Alkohol- und/oder Drogen- und/oder Medikamentenabhängigkeit und/oder Entzündungen, zur Behandlung von Depressionen, zur Vigilanzsteigerung und/oder Libidosteigerung. Ein weiterer Gegenstand der Erfindung ist die Verwendung von substituierten Aminoalkoholen der allgemeinen Formel I, vorzugsweise der allgemeinen Formel II oder III zur Herstellung von Arzneimitteln zur Behandlung von Schmerz sowie die Verwendung von substituierten Aminoalkoholen der allgemeinen Formel I, vorzugsweise der allgemeinen Formel II oder III, zur Behandlung von Emesis, cardiovaskulären Erkrankungen, Harninkontinenz, Diarrhöe, Pruritus, Alkohol- und/oder Drogen- und/oder Medikamentenabhängigkeit und/oder Entzündungen, zur Behandlung von Depressionen, zur Antriebssteigerung, Vigilanzsteigerung und/oder Libidosteigerung oder zur Herstellung eines Nootropikums (Neurotropikums).

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, formuliert und als solche auch verabreicht werden.

Neben wenigstens einer Verbindung der allgemeinen Formel I, vorzugsweise der allgemeinen Formel II oder III, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Verbindungen der allgemeinen Formel I, vorzugsweise der allgemeinen Formel II oder III, in einem Depot in gelöster Form oder in einem Pflaster, gegebenenfalls unter Zusatz von die Hautpenetration fördernden Mitteln, sind geeignete perkutane Applikationszubereitungen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen Verbindung der allgemeinen Formel I, vorzugsweise der allgemeinen Formel II oder III, kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer Verbindung der allgemeinen Formel I, vorzugsweise der allgemeinen Formel II oder III, appliziert.

Ein weiterer Gegenstand der Erfindung sind 2-(Aminomethyl)cycloalkan-1-ol-Verbindungen der allgemeinen Formel III, 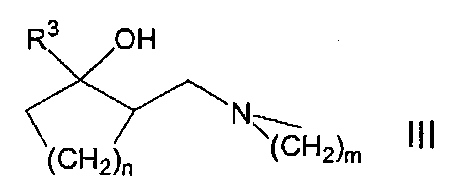 worin
n für eine ganze Zahl von 2-9 und m für 6 in folgenden Kombinationen steht,

| n | m |
|---|---|
| 2 | 6 |
| 3 | 6 |
| 4 | 6 |
| 5 | 6 |
| 9 | 6 |

wobei gegebenenfalls eine oder mehrere CH₂-Gruppen der (CH₂)ₘ-Kette durch Sauerstoff ersetzt sein können,

R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest ausgewählt aus der Gruppe umfassend Pyrrolyl-, Furyl- (Furanyl-), Thienyl- (Thiophenyl-), Pyrazolyl-, Imidazolyl-, Thiazolyl-, Oxazolyl-, Isoxazolyl-, Pyridinyl-, Pyridazinyl-, Pyrimidinyl-, Pyrazinyl- und Pyranyl steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert und/oder über eine lineare oder verzweigte, gesättigte oder ungesättigte aliphatische C₁₋₃-Brücke gebunden sein kann

in Form ihrer reinen Diastereomere, ihrer reinen Enantiomere und Gemische davon - einschließlich ihrer Racemate - sowie in Form entsprechender Basen, Salze und Solvate.

Bevorzugt sind Verbindungen der allgemeinen Formel III, worin
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann
und die übrigen Symbole die vorstehend genannte Bedeutung haben.

Ein weiterer Gegenstand der Erfindung ist ein Verfahren zur Herstellung von Verbindungen gemäß einem der Ansprüche 10-11, dadurch gekennzeichnet, daß man
A) ein Keton der Formel (1), worin n die in Anspruch 10 genannte Bedeutung hat, 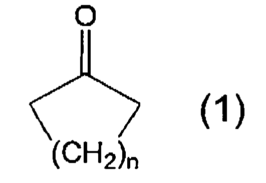
mit Paraformaldehyd und einen cyclischen Amin der Formel (2), worin m die in Anspruch 10 genannte Bedeutung hat, 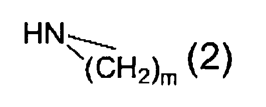 gemäß einer Mannich-Reaktion in einem geeigneten Lösungsmittel, vorzugsweise in Ethanol unter Zusatz von Salzsäure oder in Essigsäure, unter Erhitzen umsetzt, anschließend das Reaktionsgemisch aufarbeitet, das Produkt der Formel (3) isoliert und gegebenenfalls reinigt oder 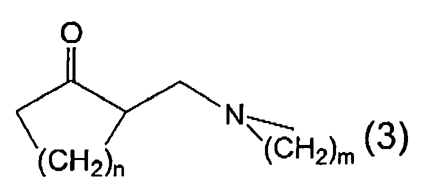
B) eine Verbindung der Formel (3) mit einer Grignard-Verbindung oder einer lithiumorganischen Verbindung der Formeln R³MgCl, R³MgBr, R³Mgl, MgR³₂ oder LiR³, worin R³ die in Anspruch 10 genannte Bedeutung hat, in einem geeigneten Lösungsmittel, vorzugsweise Diethylether oder Tetrahydrofuran, umsetzt, anschließend das Reaktionsgemisch aufarbeitet, die Verbindung der allgemeinen Formel I isoliert und gegebenenfalls reinigt.

Die zum Einsatz kommenden Ausgangsverbindungen sind am Markt erhältlich oder können nach üblichen dem Fachmann bekannten Methoden gewonnen werden.

Die Umsetzungen sind dem Fachmann aus der Literatur bekannt.

Die zum Einsatz kommenden Lösungsmittel und Umsetzungsbedindungen für die jeweilige Verfahrensstufe entsprechen den für diese Reaktionstypen üblichen Lösungsmitteln und Umsetzungsbedingungen.

Die freien Basen der jeweiligen erfindungsgemäßen Verbindungen der allgemeinen Formel III können durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden physiologisch verträglichen Salze überführt werden. Bei den gebildeten Salzen handelt es sich unter anderem um Hydrochloride, Hydrobromide, Phosphate, Carbonate, Hydrogencarbonate, Formiate, Acetate, Oxalate, Succinate, Tartrate, Fumarate, Citrate und Glutaminate.

Die freien Basen der jeweiligen erfindungsgemäßen Verbindungen der allgemeinen Formel III können durch Versetzen der in einem geeigneten organischen Lösungsmittel, wie z.B. Butan-2-on (Methylethylketon), gelösten erfindungsgemäßen Verbindungen der allgemeinen Formel I als freie Basen mit Trimethylsilylchlorid (TMSCI) in die entsprechenden Hydrochloride überführt werden. Entsprechend können sie auch in die Hydrobromide überführt werden.

Durch Kristallisation aus wäßriger Lösung können die Hydrate gebildet werden.

Sofern die erfindungsgemäßen Verbindungen der allgemeinen Formel III nach dem erfindungsgemäßen Herstellungsverfahren in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen Verbindungen der allgemeinen Formel III sind toxikologisch unbedenklich und eignen sich daher als pharmazeutische Wirkstoffe in Arzneimitteln.

### Beispiele

### Darstellung

### Mannichreaktion

Ein Äquivalent des Amin-Hydrochlorids, 1-1,5 Äquivalente des Ketons und 1-1,2 Äquivalente Paraformaldehyd in 8-10 Äquivalenten Essigsäure wurden unter Rühren 15-20 Minuten auf 95-98°C erhitzt. Anschließend wurde die Essigsäure im Vakuum schonend abdestilliert und das Reaktionsgemisch mit Aceton oder nacheinander mit Aceton und Diisopropylether versetzt Die Mannichverbindung fiel als Hydrochlorid in kristalliner Form aus.
Die Mannichreaktion konnte in einigen Fällen vorteilhaft in Ethanol, versetzt mit wenigen Tropfen konzentrierter Salzsäure, unter Rückfluß über mehrere Stunden (meist 5 bis 7 Stunden) durchgeführt werden.
Die weitere Reinigung der Mannichverbindung erfolgte, falls erforderlich, durch Kieselgel-Chromatographie mit Ethylacetat als Eluenten.

### Grignardreaktion

In einem ausgeheizten und unter Inertgas auf- 10°C abgekühlten Reaktionsgefäß wurde die in THF gelöste Mannichverbindung (400µl, 0,5 M) vorgelegt. Unter Rühren wurden daraufhin 2 Äquivalente des vorbereiteten Grignard- oder Organolithium-Reagenzes in THF oder Diethylether (800 µl, 0.5 M) zugegeben. Das Reaktionsgemisch wurde bei Raumtemperatur gerührt. Nach drei Stunden wurde erneut auf-10°C gekühlt und mit Ammoniumchlorid-Lösung hydrolisiert.
Das Reaktionsgemisch wurde zweimal mit Ethylacetat extrahiert und bei 40°C im Vakuum eingeengt.
Zur Charakterisierung des Produkts wurde ein ESI-MS aufgenommen.

### Biologische Daten

### Untersuchungen zur Noradrenalin-Wiederaufnahmehemmung (NA-Uptake-Hemmung)

Bei den erfindungsgemäßen Verbindungen handelt es sich um Inhibitoren des NA-Uptake.

Um diese in vitro Studien durchführen zu können, wurden Synaptosomen aus Rattenhirnarealen frisch isoliert. Es fand jeweils eine sogenannte "P₂"-Fraktion Verwendung, die nach der Vorschrift von Gray und Whittaker (E.G. Gray und V.P. Whittaker (1962) J. Anat. 76, 79-88) präpariert wurde. Für den NA-Uptake wurden diese vesikulären Partikel aus dem Hypothalamus männlicher Rattengehime isoliert.

### Folgende Kenndaten wurden für den NA-Transporter ermittelt:

NA-Uptake : Km = 0,32 ± 0,11 µM

(Jeweils N = 4, d.h. Mittelwerte ± SEM aus 4 unabhängigen Versuchsreihen, die in Dreifach-Parallelversuchen durchgeführt wurden).

Eine detaillierte Methodenbeschreibung kann der Litaratur entnommen werden (M.Ch. Frink, H.-H. Hennies, W. Englberger, M. Haurand und B. Wilffert (1996) Arzneim.-Forsch./Drug Res. 46 (III), 11, 1029-1036).

Von ausgewählten Verbindungen wurde die NA-Uptake-Hemmung bestimmt. Die Werte wurden bei einer Testkonzentration von 10⁻⁵ M bestimmt.

| **Beispiel** | **NA-Uptake (%-Hemmung**) |
|---|---|
| 2-Azepan-1-ylmethyl-1-(4-fluor-3-methyl-phenyl)-cyclohexanol | 54 |
| 2-Azepan-1-ylmethyl-1-(4-fluor-phenyl)-cyclohexanol | 52 |
| 2-Azepan-1-ylmethyl-1-phenethyl-cyclohexanol | 70 |
| 2-Azepan-1-ylmethyl-1-(3-phenyl-propyl)-cyclohexanol | 77 |
| 2-Azepan-1-ylmethyl-1-p-tolyl-cyclohexanol | 64 |
| 2-Azepan-1-ylmethyl-1-(4-methoxy-phenyl)-cyclohexanol | 58 |
| 2-Azepan-1-ylmethyl-1-(4-chlor-phenyl)-cyclopentanol | 80 |
| 2-Azepan-1-ylmethyl-1-(4-fluor-3-methyl-phenyl)-cyclopentanol | 73 |
| 2-Azepan-1-ylmethyl-1-m-tolyl-cyclopentanol | 59 |
| 2-Azepan-1-ylmethyl-1-phenethyl-cyclopentanol | 92 |
| 2-Azepan-1-ylmethyl-1-phenylethynyl-cyclopentanol | 99 |
| 2-Azepan-1-ylmethyl-1-thiophen-2-yl-cyclopentanol | 51 |
| 2-Azepan-1-ylmethyl-1-(3-phenyl-propyl)-cyclopentanol | 59 |
| 2-Azepan-1-ylmethyl-1-p-tolyl-cyclopentanol | 70 |
| 2-Azepan-1-ylmethyl-1-(4-methoxy-phenyl)-cyclopentanol | 58 |
| 2-Azepan-1-ylmethyl-1-phenyl-cycloheptanol | 52 |
| 2-Azepan-1-ylmethyl-1-benzyl-cycloheptanol | 55 |
| 2-Azepan-1-ylmethyl-1-(4-fluor-3-methyl-phenyl)-cycloheptanol | 63 |
| 2-Azepan-1-ylmethyl-1-phenethyl-cycloheptanol | 87 |
| 2-Azepan-1-ylmethyl-1-p-tolyl-cycloheptanol | 58 |
| 2-Azepan-1-ylmethyl-1-(4-methoxy-phenyl)-cycloheptanol | 58 |

## Patentansprüche

1. Arzneimittel enthaltend wenigstens einen substituierten Aminoalkohol der allgemeinen Formel I, 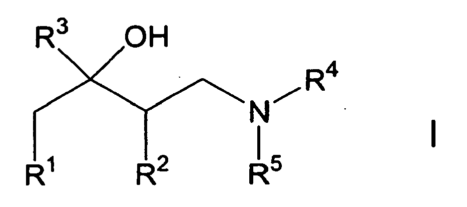 worin
R¹ und R², gleich oder verschieden, für jeweils einen linearen oder verzweigten, gesättigten oder ungesättigten aliphatischen C₁₋₃-Rest stehen oder zusammen eine (CH₂)ₙ-Kette bilden, wobei n für eine ganze Zahl von 2-9 steht,
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest ausgewählt aus der Gruppe umfassend Pyrrolyl-, Furyl- (Furanyl-), Thienyl- (Thiophenyl-), Pyrazolyl-, Imidazolyl-, Thiazolyl-, Oxazolyl-, Isoxazolyl-, Pyridinyl-, Pyridazinyl-, Pyrimidinyl-, Pyrazinyl- und Pyranyl steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert und/oder über eine lineare oder verzweigte, gesättigte oder ungesättigte aliphatische C₁₋₃-Brücke gebunden sein kann,
R⁴ und R⁵ zusammen eine (CH₂)ₘ-Kette bilden, wobei m für 6 steht und gegebenenfalls eine oder mehrere CH₂-Gruppen durch Sauerstoff ersetzt sein können,
in Form ihrer Diastereomere, ihrer Enantiomere und Gemische davon - einschließlich ihrer Racemate - sowie in Form entsprechender Basen, Salze und Solvate als Wirkstoff und gegebenenfalls physiologisch verträgliche Hilfsstoffe.

2. Arzneimittel enthaltend wenigstens eine Verbindung gemäß Anspruch 1, **dadurch gekennzeichnet, daß**
R¹ und R² jeweils für eine Methylgruppe stehen oder zusammen eine (CH₂)ₙ-Kette bilden, wobei n für 2, 3, 4, 5 oder 9 steht,
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann,
R⁴ und R⁵ zusammen eine (CH₂)ₘ-Kette bilden, wobei m für 6 steht.

3. Arzneimittel enthaltend wenigstens eine Verbindung gemäß Anspruch 1 entsprechend der allgemeinen Formel II, 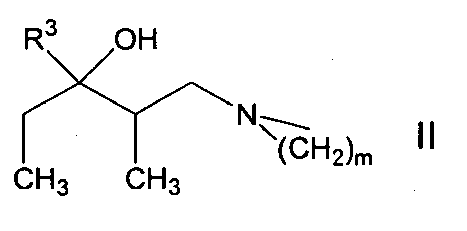 worin
m für 6 steht.

4. Arzneimittel enthaltend wenigstens eine Verbindung gemäß Anspruch 1 entsprechend der allgemeinen Formel III, 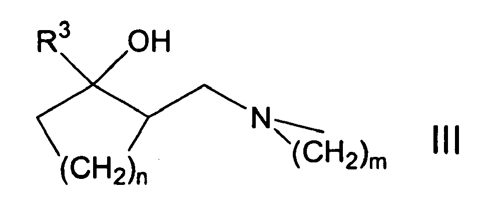 worin
n für eine ganze Zahl von 2-9 und m für 6 stehen.

5. Arzneimittel enthaltend wenigstens eine Verbindung gemäß Anspruch 3 oder 4, **dadurch gekennzeichnet, daß**
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest ausgewählt aus der Gruppe umfassend Pyrrolyl-, Furyl- (Furanyl-), Thienyl- (Thiophenyl-), Pyrazolyl-, Imidazolyl-, Thiazolyl-, Oxazolyl-, Isoxazolyl-, Pyridinyl-, Pyridazinyl-, Pyrimidinyl-, Pyrazinyl- und Pyranyl steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert und/oder über eine lineare oder verzweigte, gesättigte oder ungesättigte aliphatische C₁₋₃-Brücke gebunden sein kann.

6. Arzneimittel enthaltend wenigstens eine Verbindung gemäß einem der Ansprüche 3 oder 4, **dadurch gekennzeichnet, daß**
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann.

7. Arzneimittel enthaltend wenigstens einen substituierten Aminoalkohol gemäß Anspruch 1 ausgewählt aus der Gruppe umfassend
2-Azepan-1-ylmethyl-1-phenyl-cyclohexanol
2-Azepan-1-ylmethyl-1-benzyl-cyclohexanol
2-Azepan-1-ylmethyl-1-(4-fluor-3-methyl-phenyl)-cyclohexanol
2-Azepan-1-ylmethyl-1-o-tolyl-cyclohexanol
2-Azepan-1-ylmethyl-1-vinyl-cyclohexanol
2-Azepan-1-ylmethyl-1-(4-tert-butyl-phenyl)-cyclohexanol
2-Azepan-1-ylmethyl-1-cyclopentyl-cyclohexanol
2-Azepan-1-ylmethyl-1-m-tolyl-cyclohexanol
2-Azepan-1-ylmethyl-bicyclohexyl-1-ol
2-Azepan-1-ylmethyl-1-(4-fluor-phenyl)-cyclohexanol
2-Azepan-1-ylmethyl-1-phenethyl-cyclohexanol
2-Azepan-1-ylmethyl-1-thiophen-2-yl-cyclohexanol
2-Azepan-1-ylmethyl-1-(2,4-dichlor-phenyl)-cyclohexanol
2-Azepan-1-ylmethyl-1-(3-methoxy-phenyl)-cyclohexanol
2-Azepan-1-ylmethyl-1-(3-phenyl-propyl)-cyclohexanol
2-Azepan-1-ylmethyl-1-p-tolyl-cyclohexanol
2-Azepan-1-ylmethyl-1-(4-methoxy-phenyl)-cyclohexanol
2-Azepan-1-ylmethyl-1-phenyl-cyclopentanol
2-Azepan-1-ylmethyl-1-(4-chlor-phenyl)-cyclopentanol
2-Azepan-1-ylmethyl-1-benzyl-cyclopentanol
2-Azepan-1-ylmethyl-1-(4-fluor-3-methyl-phenyl)-cyclopentanol
2-Azepan-1-ylmethyl-1-o-tolyl-cyclopentanol
2-Azepan-1-ylmethyl-1-vinyl-cyclopentanol
2-Azepan-1-ylmethyl-1-(4-tert-butyl-phenyl)-cyclopentanol
2-Azepan-1-ylmethyl-bicyclopentyl-1-ol
2-Azepan-1-ylmethyl-1-m-tolyl-cyclopentanol
2-Azepan-1-ylmethyl-1-cyclohexyl-cyclopentanol
2-Azepan-1-ylmethyl-1-(4-fluor-phenyl)-cyclopentanol
2-Azepan-1-ylmethyl-1-phenethyl-cyclopentanol
2-Azepan-1-ylmethyl-1-phenylethynyl-cyclopentanol
2-Azepan-1-ylmethyl-1-thiophen-2-yl-cyclopentanol
2-Azepan-1-ylmethyl-1-(3-methoxy-phenyl)-cyclopentanol
2-Azepan-1-ylmethyl-1-(3-phenyl-propyl)-cyclopentanol
2-Azepan-1-ylmethyl-1-p-tolyl-cyclopentanol
2-Azepan-1-ylmethyl-1-(4-methoxy-phenyl)-cyclopentanol
2-Azepan-1-ylmethyl-1-phenyl-cycloheptanol
2-Azepan-1-ylmethyl-1-benzyl-cycloheptanol
2-Azepan-1-ylmethyl-1-(4-fluor-3-methyl-phenyl)-cycloheptanol
2-Azepan-1-ylmethyl-1-o-tolyl-cycloheptanol
2-Azepan-1-ylmethyl-1-vinyl-cycloheptanol
2-Azepan-1-ylmethyl-1-(4-tert-buty)-phenyl)-cycloheptanol
2-Azepan-1-ylmethyl-1-cyclopentyl-cycloheptanol
2-Azepan-1-ylmethyl-1-m-tolyl-cycloheptanol
2-Azepan-1-ylmethyl-1-cyclohexyl-cycloheptanol
2-Azepan-1-ylmethyl-1-(4-fluor-phenyl)-cycloheptanol
2-Azepan-1-ylmethyl-1-phenethyl-cycloheptanol
2-Azepan-1-ylmethyl-1-phenylethynyl-cycloheptanol
2-Azepan-1-ylmethyl-1-thiophen-2-yl-cycloheptanol
2-Azepan-1-ylmethyl-1-(2,4-dichlor-phenyl)-cycloheptanol
2-Azepan-1-ylmethyl-1-(3-methoxy-phenyl)-cycloheptanol
2-Azepan-1-ylmethyl-1-(3-phenyl-propyl)-cycloheptanol
2-Azepan-1-ylmethyl-1-p-tolyl-cycloheptanol,
2-Azepan-1-ylmethyl-1-(4-methoxy-phenyl)-cycloheptanol
2-Azepan-1-ylmethyl-1-(3-phenyl-propyl)-cyclopentanol
2-Azepan-1-ylmethyl-1-(2,3-dichlor-phenyl)-cyclopentanol
2-Azepan-1-ylmethyl-1-benzyl-cyclododecanol
2-Azepan-1-ylmethyl-1-vinyl-cyclododecanol
2-Azepan-1-ylmethyl-1-1-(4-tert-butyl-phenyl)-cyclododecanol
2-Azepan-1-ylmethyl-1-phenethyl-cyclododecanol
2-Azepan-1-ylmethyl-1-(3-phenyl-propyl)-cyclododecanol
2-Azepan-1-ylmethyl-1-(3-fluor-phenyl)-cyclohexanol
2-Azepan-1-ylmethyl-1-(3-chlor-phenyl)-cyclohexanol
2-Azepan-1-ylmethyl-1-cyclohexylmethyl-cyclopentanol
2-Azepan-1-ylmethyl-1-(2-chlor-6-fluor-benzyl)-cyclopentanol
2-Azepan-1-ylmethyl-1-(2,5-dimethyl-benzyl)-cyclopentanol
2-Azepan-1-ylmethyl-1-(4-fluor-benzyl)-cycloheptanol
1-Azepan-1-yl-3-cyclohexylmethyl-2-methyl-pentan-3-ol
1-Azepan-1-yl-3-(5-fluor-2-methoxy-phenyl)-2-methyl-pentan-3-ol
2-Azepan-1-ylmethyl-1-(3-methoxy-benzyl)-cycloheptanol
2-Azepan-1-ylmethyl-1-(3-methoxy-benzyl)-cycloheptanol
2-Azepan-1-ylmethyl-1-(3-fluor-benzyl)-cyclopentanol
2-Azepan-1-ylmethyl-1-(3-phenyl-propyl)-cyclooctanol
in Form ihrer Diastereomeren, ihrer Enantiomere und Gemische davon - einschließlich ihrer Racemate - sowie in Form entsprechender Basen, Salze und Solvate
und gegebenenfalls physiologisch verträgliche Hilfsstoffe.

8. Verwendung wenigstens einer Verbindung gemäß einem der Ansprüche 1-7 zur Herstellung eines Arzneimittels zur Behandlung von Schmerz.

9. Verwendung wenigstens einer Verbindung gemäß einem der Ansprüche 1-7 zur Herstellung eines Arzneimittels zur Behandlung von Emesis, cardiovaskulären Erkrankungen, Harninkontinenz, Diarrhöe, Pruritus, Alkohol- und/oder Drogen- und/oder Medikamentenabhängigkeit und/oder Entzündungen, zur Behandlung von Depressionen, zur Antriebssteigerung, Vigilanzsteigerung und/oder Libidosteigerung oder zur Herstellung eines Nootropikums (Neurotropikums).

10. 2-(Aminomethyl)cycloalkan-1-ol-Verbindungen der allgemeinen Formel III, 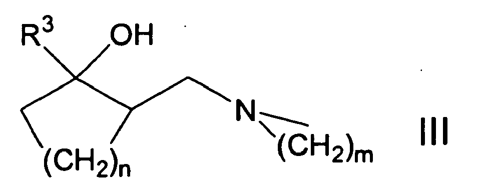 worin
n für eine ganze Zahl von 2-9 und m für 6 in folgenden Kombinationen steht,
| n | m |
|---|---|
| 2 | 6 |
| 3 | 6 |
| 4 | 6 |
| 5 | 6 |
| 9 | 6 |
wobei gegebenenfalls eine oder mehrere CH₂-Gruppen der (CH₂)ₘ-Kette durch Sauerstoff ersetzt sein können,
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest ausgewählt aus der Gruppe umfassend Pyrrolyl-, Furyl- (Furanyl-), Thienyl- (Thiophenyl-), Pyrazolyl-, Imidazolyl-, Thiazolyl-, Oxazolyl-, Isoxazolyl-, Pyridinyl-, Pyridazinyl-, Pyrimidinyl-, Pyrazinyl- und Pyranyl steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert und/oder über eine lineare oder verzweigte, gesättigte oder ungesättigte aliphatische C₁₋₃-Brücke gebunden sein kann
in Form ihrer reinen Diastereomere, ihrer reinen Enantiomere und Gemische davon - einschließlich ihrer Racemate - sowie in Form entsprechender Basen, Salze und Solvate.

11. Verbindungen gemäß Anspruch 10, **dadurch gekennzeichnet, daß**
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann.

12. Verfahren zur Herstellung von Verbindungen gemäß einem der Ansprüche 10-11, **dadurch gekennzeichnet, daß** man
A) ein Keton der Formel (1), worin n die in Anspruch 10 genannte Bedeutung hat, 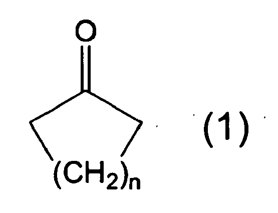 mit Paraformaldehyd und einen cyclischen Amin der Formel (2), worin m die in Anspruch 10 genannte Bedeutung hat, 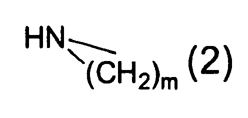 gemäß einer Mannich-Reaktion in einem geeigneten Lösungsmittel, vorzugsweise in Ethanol unter Zusatz von Salzsäure oder in Essigsäure, unter Erhitzen umsetzt, anschließend das Reaktionsgemisch aufarbeitet, das Produkt der Formel (3) isoliert und gegebenenfalls reinigt oder 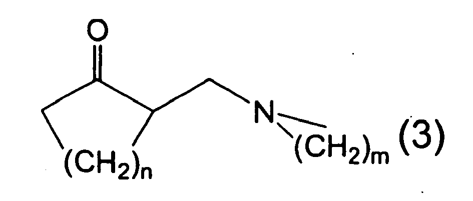
B) eine Verbindung der Formel (3) mit einer Grignard-Verbindung oder einer lithiumorganischen Verbindung der Formeln R³MgCl, R³MgBr, R³Mgl, MgR³₂ oder LiR³, worin R³ die in Anspruch 10 genannte Bedeutung hat, in einem geeigneten Lösungsmittel, vorzugsweise Diethylether oder Tetrahydrofuran, umsetzt, anschließend das Reaktionsgemisch aufarbeitet, die Verbindung der allgemeinen Formel I isoliert und gegebenenfalls reinigt.

## Claims

1. A medicament containing at least one substituted aminoalcohol of the general formula I, 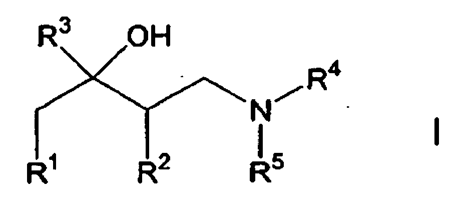 in which
R¹ and R², identical or different, in each case denote a linear or branched, saturated or unsaturated aliphatic C₁₋₃ residue, or together form a (CH₂)ₙ chain, wherein n denotes an integer from 2-9,
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue selected from the group comprising pyrrolyl, furyl (furanyl), thienyl (thiophenyl), pyrazolyl, imidazolyl, thiazolyl, oxazolyl, isoxazolyl, pyridinyl, pyridazinyl, pyrimidinyl, pyrazinyl and pyranyl, wherein the respective ring system may optionally be mono- or polysubstituted with halogen, an alkyl group, an alkoxy group and/or a trihalogenated alkyl group and/or may be attached via a linear or branched, saturated or unsaturated aliphatic C₁₋₃ bridge,
R⁴ and R⁵ together form a (CH₂)ₘ chain, wherein m denotes 6 and optionally one or more CH₂ groups may be replaced by oxygen,
in the form of the diastereomers thereof, the enantiomers thereof and mixtures thereof - including the racemates thereof - and in the form of corresponding bases, salts and solvates as active ingredient and optionally physiologically acceptable auxiliary substances.

2. A medicament containing at least one compound according to claim 1,
**characterised in that**
R¹ and R² in each case denote a methyl group, or together form a (CH₂)ₙ chain, wherein n denotes 2, 3, 4, 5 or 9,
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge,
R⁴ and R⁵ together form a (CH₂)ₘ chain, wherein m denotes 6.

3. A medicament containing at least one compound according to claim 1 corresponding to the general formula II, 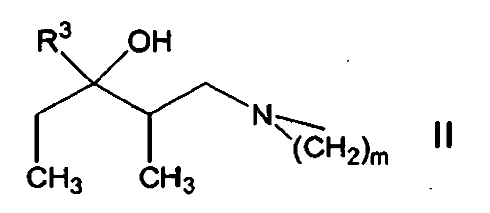 in which
m denotes 6.

4. A medicament containing at least one compound according to claim 1 corresponding to the general formula III, 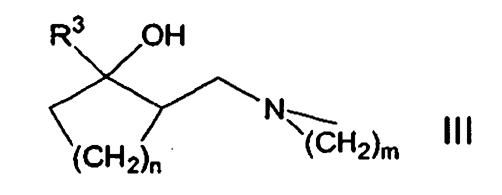 in which
n denotes an integer from 2-9 and m denotes 6.

5. A medicament containing at least one compound according to claim 3 or claim 4, or **characterised in that**
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue selected from the group comprising pyrrolyl, furyl (furanyl), thienyl (thiophenyl), pyrazolyl, imidazolyl, thiazolyl, oxazolyl, isoxazolyl, pyridinyl, pyridazinyl, pyrimidinyl, pyrazinyl and pyranyl, wherein the respective ring system may optionally be mono- or polysubstituted with halogen, an alkyl group, an alkoxy group and/or a trihalogenated alkyl group and/or may be attached via a linear or branched, saturated or unsaturated aliphatic C₁₋₃ bridge.

6. A medicament containing at least one compound according to either one of claims 3 or 4, **characterised in that**
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge.

7. A medicament containing at least one substituted aminoalcohol according to claim 1 selected from the group comprising
2-azepan-1-ylmethyl-1-phenyl-cyclohexanol
2-azepan-1-ylmethyl-1-benzyl-cyclohexanol
2-azepan-1-ylmethyl-1-(4-fluoro-3-methyl-phenyl)-cyclohexanol
2-azepan-1-ylmethyl-1-o-tolyl-cyclohexanol
2-azepan-1-ylmethyl-1-vinyl-cyclohexanol
2-azepan-1-ylmethyl-1-(4-tert-butyl-phenyl)-cyclohexanol
2-azepan-1-ylmethyl-1-cyclopentyl-cyclohexanol
2-azepan-1-ylmethyl-1-m-tolyl-cyclohexanol
2-azepan-1-ylmethyl-bicyclohexyl-1-ol
2-azepan-1-ylmethyl-1-(4-fluoro-phenyl)-cyclohexanol
2-azepan-1-ylmethyl-1-phenethyl-cyclohexanol
2-azepan-1-ylmethyl-1-thiophen-2-yl-cyclohexanol
2-azepan-1-ylmethyl-1-(2,4-dichloro-phenyl)-cyclohexanol
2-azepan-1-ylmethyl-1-(3-methoxy-phenyl)-cyclohexanol
2-azepan-1-ylmethyl-1-(3-phenyl-propyl)-cyclohexanol
2-azepan-1-ylmethyl-1-p-tolyl-cyclohexanol
2-azepan-1-ylmethyl-1-(4-methoxy-phenyl)-cyclohexanol
2-azepan-1-ylmethyl-1-phenyl-cyclopentanol
2-azepan-1-ylmethyl-1-(4-chloro-phenyl)-cyclopentanol
2-azepan-1-ylmethyl-1-benzyl-cyclopentanol
2-azepan-1-ylmethyl-1-(4-fluoro-3-methyl-phenyl)-cyclopentanol
2-azepan-1-ylmethyl-1-o-tolyl-cyclopentanol
2-azepan-1-ylmethyl-1-vinyl-cyclopentanol
2-azepan-1-ylmethyl-1-(4-tert-butyl-phenyl)-cyclopentanol
2-azepan-1-ylmethyl-bicyclopentyl-1-ol
2-azepan-1-ylmethyl-1-m-tolyl-cyclopentanol
2-azepan-1-ylmethyl-1-cyclohexyl-cyclopentanol
2-azepan-1-ylmethyl-1-(4-fluoro-phenyl)-cyclopentanol
2-azepan-1-ylmethyl-1-phenethyl-cyclopentanol
2-azepan-1-ylmethyl-1-phenylethynyl-cyclopentanol
2-azepan-1-ylmethyl-1-thiophen-2-yl-cyclopentanol
2-azepan-1-ylmethyl-1-(3-methoxy-phenyl)-cyclopentanol
2-azepan-1-ylmethyl-1-(3-phenyl-propyl)-cyclopentanol
2-azepan-1-ylmethyl-1-p-tolyl-cyclopentanol
2-azepan-1-ylmethyl-1-(4-methoxy-phenyl)-cyclopentanol
2-azepan-1-ylmethyl-1-phenyl-cycloheptanol
2-azepan-1-ylmethyl-1-benzyl-cycloheptanol
2-azepan-1-ylmethyl-1-(4-fluoro-3-methyl-phenyl)-cycloheptanol
2-azepan-1-ylmethyl-1-o-tolyl-cycloheptanol
2-azepan-1-ylmethyl-1-vinyl-cycloheptanol
2-azepan-1-ylmethyl-1-(4-tert-butyl-phenyl)-cycloheptanol
2-azepan-1-ylmethyl-1-cyclopentyl-cycloheptanol
2-azepan-1-ylmethyl-1-m-tolyl-cycloheptanol
2-azepan-1-ylmethyl-1-cyclohexyl-cycloheptanol
2-azepan-1-ylmethyl-1-(4-fluoro-phenyl)-cycloheptanol
2-azepan-1-ylmethyl-1-phenethyl-cycloheptanol
2-azepan-1-ylmethyl-1-phenylethynyl-cycloheptanol
2-azepan-1-ylmethyl-1-thiophen-2-yl-cycloheptanol
2-azepan-1-ylmethyl-1-(2,4-dichloro-phenyl)-cycloheptanol
2-azepan-1-ylmethyl-1-(3-methoxy-phenyl)-cycloheptanol
2-azepan-1-ylmethyl-1-(3-phenyl-propyl)-cycloheptanol
2-azepan-1-ylmethyl-1-p-tolyl-cycloheptanol
2-azepan-1-ylmethyl-1-(4-methoxy-phenyl)-cycloheptanol
2-azepan-1-ylmethyl-1-(3-phenyl-propyl)-cyclopentanol
2-azepan-1-ylmethyl-1-(2,3-dichloro-phenyl)-cyclopentanol
2-azepan-1-ylmethyl-1-benzyl-cyclododecanol
2-azepan-1-ylmethyl-1-vinyl-cyclododecanol
2-azepan-1-ylmethyl-1-(4-tert-butyl-phenyl)-cyclododecanol
2-azepan-1-ylmethyl-1-phenethyl-cyclododecanol
2-azepan-1-ylmethyl-1-(3-phenyl-propyl)-cyclododecanol
2-azepan-1-ylmethyl-1-(3-fluoro-phenyl)-cyclohexanol
2-azepan-1-ylmethyl-1-(3-chloro-phenyl)-cyclohexanol
2-azepan-1-ylmethyl-1-cyclohexylmethyl-cyclopentanol
2-azepan-1-ylmethyl-1-(2-chloro-6-fluoro-benzyl)-cyclopentanol
2-azepan-1-ylmethyl-1-(2,5-dimethyl-benzyl)-cyclopentanol
2-azepan-1-ylmethyl-1-(4-fluoro-benzyl)-cycloheptanol
1-azepan-1-yl-3-cyclohexylmethyl-2-methyl-pentan-3-ol
1-azepan-1-yl-3-(5-fluoro-2-methoxy-phenyl)-2-methyl-pentan-3-ol
2-azepan-1-ylmethyl-1-(3-methoxy-benzyl)-cycloheptanol
2-azepan-1-ylmethyl-1-(3-methoxy-benzyl)-cycloheptanol
2-azepan-1-ylmethyl-1-(3-fluoro-benzyl)-cyclopentanol
2-azepan-1-ylmethyl-1-(3-phenyl-propyl)-cyclooctanol
in the form of the diastereomers thereof, the enantiomers thereof and mixtures thereof - including the racemates thereof - and in the form of corresponding bases, salts and solvates and optionally physiologically acceptable auxiliary substances.

8. Use of at least one compound according to one of claims 1-7 for the production of a medicament for the treatment of pain.

9. Use of at least one compound according to one of claims 1-7 for the production of a medicament for the treatment of emesis, cardiovascular diseases, urinary incontinence, diarrhoea, pruritus, dependency on alcohol and/or drugs and/or medicines and/or inflammation, for the treatment of depression, to increase drive, vigilance and/or libido or for the production of a nootropic (neurotropic).

10. 2-(Aminomethyl)-cycloalkan-1-ol compounds of the general formula III, 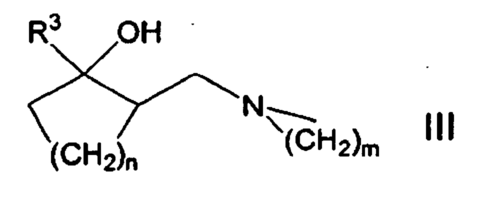 in which
n denotes an integer from 2-9 and m denotes 6 in the following combinations,
| n | m |
|---|---|
| 2 | 6 |
| 3 | 6 |
| 4 | 6 |
| 5 | 6 |
| 9 | 6 |
wherein optionally one or more CH₂ groups of the (CH₂)ₘ chain may be replaced by oxygen,
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue selected from the group comprising pyrrolyl, furyl (furanyl), thienyl (thiophenyl), pyrazolyl, imidazolyl, thiazolyl, oxazolyl, isoxazolyl, pyridinyl, pyridazinyl, pyrimidinyl, pyrazinyl and pyranyl, wherein the respective ring system may optionally be mono- or polysubstituted with halogen, an alkyl group, an alkoxy group and/or a trihalogenated alkyl group and/or may be attached via a linear or branched, saturated or unsaturated aliphatic C₁₋₃ bridge
in the form of the pure diastereomers thereof, the pure enantiomers thereof and mixtures thereof - including the racemates thereof - and in the form of corresponding bases, salts and solvates.

11. Compounds according to claim 10, **characterised in that**
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge.

12. A process for the production of compounds according to one of claims 10-11, **characterised in that**
A) a ketone of the formula (1), in which n has the meaning stated in claim 10, 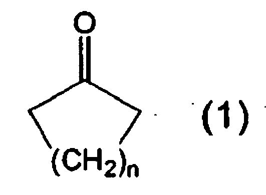 is reacted with paraformaldehyde and a cyclic amine of the formula (2), in which m has the meaning stated in claim 10, 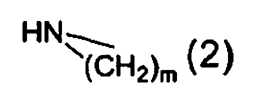 according to a Mannich reaction in a suitable solvent, preferably in ethanol with the addition of hydrochloric acid or in acetic acid, with heating, then the reaction mixture is worked up and the product of the formula (3) is isolated and optionally purified or 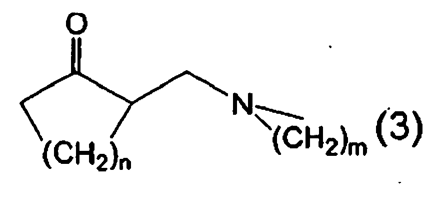
B) a compound of the formula (3) is reacted with a Grignard compound or an organolithium compound of the formulae R³MgCl,
R³MgBᵣ, R³Mgl, MgR³₂ or LiR³, in which R³ has the meaning stated in claim 10, in a suitable solvent, preferably diethyl ether or tetrahydrofuran, then the reaction mixture is worked up and the compound of the general formula I is isolated and optionally purified.

## Revendications

1. Médicament contenant au moins un aminoalcool substitué de formule générale I, 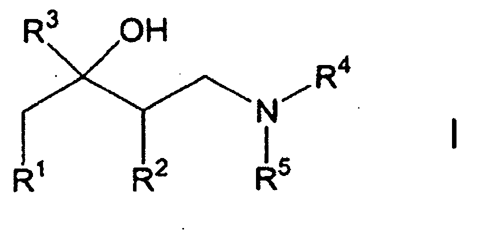 dans laquelle
R¹ et R², identiques ou différents, représentent chacun un radical aliphatique en C₁-C₃ linéaire ou ramifié, saturé ou insaturé, ou forment ensemble une chaîne (CH₂)ₙ, n représentant un nombre entier allant de 2 à 9,
R³ représente un radical aliphatique en C₁-C₃ linéaire ou ramifié, saturé ou insaturé, un radical cycloaliphatique en C₅-C₆ saturé ou insaturé, un radical phényle ou un radical hétéroaryle à cinq ou six chaînons, choisi dans l'ensemble comprenant les groupes pyrrolyle, furyle (furannyle), thiényle (thiophényle, pyrazolyle, imidazolyle, thiazolyle, oxazolyle, isoxazolyle, pyridinyle, pyridazinyle, pyrimidinyle, pyrazinyle et pyrannyle, le système cyclique respectif pouvant éventuellement être une ou plusieurs fois substitué par un atome d'halogène ou par un groupe alkyle, par un groupe alcoxy et/ou par un groupe alkyle trihalogéné et/ou être lié par un pont aliphatique en C₁-C₃ linéaire ou ramifié, saturé ou insaturé,
R⁴ et R⁵ forment ensemble une chaîne (CH₂)ₘ, m représentant 6 et un ou plusieurs groupes CH₂ pouvant éventuellement être remplacés par un/des atomes d'oxygène,
sous forme de leurs diastéréoisomères, de leurs énantiomères et de mélanges de ceux-ci - y compris de leurs racémates - ainsi que sous forme de bases correspondantes, de sels correspondants et de produits de solvatation correspondants, en tant que substance active, et éventuellement des adjuvants physiologiquement acceptables.

2. Médicament contenant au moins un composé selon la revendication 1, **caractérisé en ce que**
R¹ et R² représentent chacun un group méthyle ou forment ensemble une chaîne (CH₂)ₙ, n représentant le nombre 2, 3, 4, 5 ou 9,
R³ représente un radical vinyle, un radical cyclopentyle, un radical cyclohexyle, un radical thiophényle ou un radical phényle, le radical cyclohexyle pouvant éventuellement être lié par un pont méthylène ou le radical phényle pouvant éventuellement être une ou plusieurs fois substitué par un atome de fluor, de chlore, par un groupe méthyle, un groupe isopropyle, un groupe méthoxy et/ou un groupe trifluorométhyle et/ou éventuellement être lié par un pont aliphatique en C₁-C₃ linéaire, saturé, ou par un pont éthynyle,
R⁴ et R⁵ forment ensemble une chaîne (CH₂)ₘ, m représentant 6.

3. Médicament contenant au moins un composé selon la revendication 1, correspondant à la formule générale II, 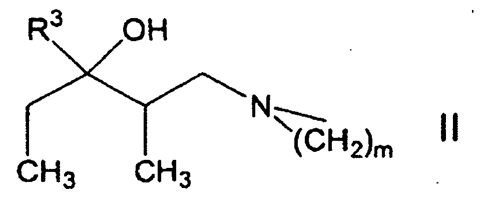 dans laquelle
m représente 6.

4. Médicament contenant au moins un composé selon la revendication 1, correspondant à la formule générale III, 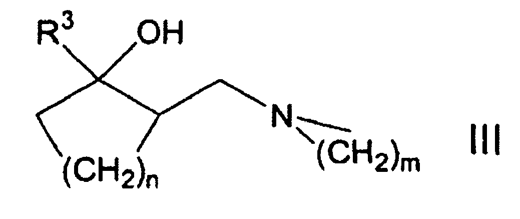 dans laquelle
n représente un nombre entier allant de 2 à 9 et m est égal à 6.

5. Médicament contenant au moins un composé selon la revendication 3 ou 4, **caractérisé en ce que**
R³ représente un radical aliphatique en C₁-C₃ linéaire ou ramifié, saturé ou insaturé, un radical cycloaliphatique en C₅-C₆ saturé ou insaturé, un radical phényle ou un radical hétéroaryle à cinq ou six chaînons, choisi dans l'ensemble comprenant les groupes pyrrolyle, furyle (furannyle), thiényle (thiophényle, pyrazolyle, imidazolyle, thiazolyle, oxazolyle, isoxazolyle, pyridinyle, pyridazinyle, pyrimidinyle, pyrazinyle et pyrannyle, le système cyclique respectif pouvant éventuellement être une ou plusieurs fois substitué par un atome d'halogène ou par un groupe alkyle, par un groupe alcoxy et/ou par un groupe alkyle trihalogéné et/ou être lié par un pont aliphatique en C₁-C₃ linéaire ou ramifié, saturé ou insaturé,

6. Médicament contenant au moins un composé selon la revendication 3 ou 4, **caractérisé en ce que**
R³ représente un radical vinyle, un radical cyclopentyle, un radical cyclohexyle, un radical thiophényle ou un radical phényle, le radical cyclohexyle pouvant éventuellement être lié par un pont méthylène ou le radical phényle pouvant éventuellement être une ou plusieurs fois substitué par un atome de fluor, de chlore, par un groupe méthyle, un groupe isopropyle, un groupe méthoxy et/ou un groupe trifluorométhyle et/ou éventuellement être lié par un pont aliphatique en C₁-C₃ linéaire, saturé, ou par un pont éthynyle,

7. Médicament contenant au moins un aminoalcool substitué selon la revendication 1, choisi dans le groupe comprenant
le 2-azépan-1-ylméthyl-1-phényl-cyclohexanol
le 2-azépan-1-ylméthyl-1-benzyl-cyclohexanol
le 2-azépan-1-ylméthyl-1-(4-fluoro-3-méthyl-phényl)-cyclohexanol
le 2-azépan-1-ylméthyl-1-o-tolyl-cyclohexanol
le 2-azépan-1-ylméthyl-1-vinyl-cyclohexanol
le 2-azépan-1-ylméthyl-1-(4-tert-butyl-phényl)-cyclohexanol
le 2-azépan-1-ylméthyl-1-cyclopentyl-cyclohexanol
le 2-azépan-1-ylméthyl-1-m-tolyl-cyclohexanol
le 2-azépan-1-ylméthyl-bicyclohexyl-1-ol
le 2-azépan-1-ylméthyl-1-(4-fluor-phényl)-cyclohexanol
le 2-azépan-1-ylméthyl-1-phenéthyl-cyclohexanol
le 2-azépan-1-ylméthyl-1-thiophén-2-yl-cyclohexanol
le 2-azépan-1-ylméthyl-1-(2,4-dichloro-phényl)-cyclohexanol
le 2-azépan-1-ylméthyl-1-(3-méthoxy-phényl)-cyclohexanol
le 2-azépan-1-ylméthyl-1-(3-phényl-propyl)-cyclohexanol
le 2-azépan-1-ylméthyl-1-p-tolyl-cyclohexanol
le 2-azépan-1-ylméthyl-1-(4-méthoxy-phényl)-cyclohexanol
le 2-azépan-1-ylméthyl-1-phényl-cyclopentanol
le 2-azépan-1-ylméthyl-1-(4-chloro-phényl)-cyclopentanol
le 2-azépan-1-ylméthyl-1-benzyl-cyclopentanol
le 2-azépan-1-ylméthyl-1-(4-fluoro-3-méthyl-phényl)-cyclopentanol
le 2-azépan-1-ylméthyl-1-o-tolyl-cyclopentanol
le 2-azépan-1-ylméthyl-1-vinyl-cyclopentanol
le 2-azépan-1-ylméthyl-1-(4-tert-butyl-phényl)-cyclopentanol
le 2-azépan-1-ylméthyl-bicyclopentyl-1-ol
le 2-azépan-1-ylméthyl-1-m-tolyl-cyclopentanol
le 2-azépan-1-ylméthyl-1-cyclohexyl-cyclopentanol
le 2-azépan-1-ylméthyl-1-(4-fluoro-phényl)-cyclopentanol
le 2-azépan-1-ylméthyl-1-phenéthyl-cyclopentanol
le 2-azépan-1-ylméthyl-1-phényléthynyl-cyclopentanol
le 2-azépan-1-ylméthyl-1-thiophén-2-yl-cyclopentanol
le 2-azépan-1-ylméthyl-1-(3-méthoxy-phényl)-cyclopentanol
le 2-azépan-1-ylméthyl-1-(3-phényl-propyl)-cyclopentanol
le 2-azépan-1-ylméthyl-1-p-tolyl-cyclopentanol
le 2-azépan-1-ylméthyl-1-(4-méthoxy-phényl)-cyclopentanol
le 2-azépan-1-ylméthyl-1-phényl-cycloheptanol
le 2-azépan-1-ylméthyl-1-benzyl-cycloheptanol
le 2-azépan-1-ylméthyl-1-(4-fluoro-3-méthyl-phényl)-cycloheptanol
le 2-azépan-1-ylméthyl-1-o-tolyl-cycloheptanol
le 2-azépan-1-ylméthyl-1-vinyl-cycloheptanol
le 2-azépan-1-ylméthyl-1-(4-tert-butyl-phényl)-cycloheptanol
le 2-azépan-1-ylméthyl-1-cyclopentyl-cycloheptanol
le 2-azépan-1-ylméthyl-1-m-tolyl-cycloheptanol
le 2-azépan-1-ylméthyl-1-cyclohexyl-cycloheptanol
le 2-azépan-1-ylméthyl-1-(4-fluor-phényl)-cycloheptanol
le 2-azépan-1-ylméthyl-1-phenéthyl-cycloheptanol
le 2-azépan-1-ylméthyl-1-phényléthynyl-cycloheptanol
le 2-azépan-1-ylméthyl-1-thiophén-2-yl-cycloheptanol
le 2-azépan-1-ylméthyl-1-(2,4-dichloro-phényl)-cycloheptanol
le 2-azépan-1-ylméthyl-1-(3-méthoxy-phényl)-cycloheptanol
le 2-azépan-1-ylméthyl-1-(3-phényl-propyl)-cycloheptanol
le 2-azépan-1-ylméthyl-1-p-tolyl-cycloheptanol
le 2-azépan-1-ylméthyl-1-(4-méthoxy-phényl)-cycloheptanol
le 2-azépan-1-ylméthyl-1-(3-phényl-propyl)-cyclopentanol
le 2-azépan-1-ylméthyl-1-(2,3-dichloro-phényl)-cyclopentanol
le 2-azépan-1-ylméthyl-1-benzyl-cyclododécanol
le 2-azépan-1-ylméthyl-1-vinyl-cyclododécanol
le 2-azépan-1-ylméthyl-1-(4-tert-butyl-phényl)-cyclododécanol
le 2-azépan-1-ylméthyl-1-phenéthyl-cyclododécanol
le 2-azépan-1-ylméthyl-1-(3-phényl-propyl)-cyclododécanol
le 2-azépan-1-ylméthyl-1-(3-fluoro-phényl)-cyclohexanol
le 2-azépan-1-ylméthyl-1-(3-chloro-phényl)-cyclohexanol
le 2-azépan-1-ylméthyl-1-cyclohexylméthyl-cyclopentanol
le 2-azépan-1-ylméthyl-1-(2-chloro-6-fluor-benzyl)-cyclopentanol
le 2-azépan-1-ylméthyl-1-(2,5-diméthyl-benzyl)-cyclopentanol
le 2-azépan-1-ylméthyl-1-(4-fluor-benzyl)-cycloheptanol
le 1-azépan-yl-3-cyclohexylméthyl-2-méthyl-pentan-3-ol
le 1-azépan-yl-3-(5-fluor-2-méthoxy-phényl)-2-méthylpentan-3-ol
le 2-azépan-1-ylméthyl-1-(3-méthoxy-benzyl)-cycloheptanol
le 2-azépan-1-ylméthyl-1-(3-méthoxy-benzyl)-cycloheptanol
le 2-azépan-1-ylméthyl-1-(3-fluor-benzyl)-cyclopentanol
le 2-azépan-1-ylméthyl-1-(3-phényl-propyl)-cyclooctanol
sous forme de leurs diastéréoisomères, de leurs énantiomères et de mélanges de ceux-ci - y compris de leurs racémates - ainsi que sous forme de bases correspondantes, de sels correspondants et de produits de solvatation correspondants
et éventuellement des adjuvants physiologiquement acceptables.

8. Utilisation d'au moins un composé selon l'une quelconque des revendications 1 à 7, pour la fabrication d'un médicament destiné au traitement de la douleur.

9. Utilisation d'au moins un composé selon l'une quelconque des revendications 1 à 7, pour la fabrication d'un médicament destiné au traitement des vomissements, des maladies cardiovasculaires, de l'incontinence urinaire, de la diarrhée, du prurit, de la dépendance à l'alcool et/ou aux drogues et/ou de la pharmacodépendance et/ou d'inflammations, au traitement de dépressions, à l'augmentation du tonus, à l'accroissement de la vigilance et/ou à l'accroissement de la libido ou pour la fabrication d'un produit nootrope (neurotrope).

10. Composés 2-(aminométhyl)cycloalcan-1-ol de formule générale III, 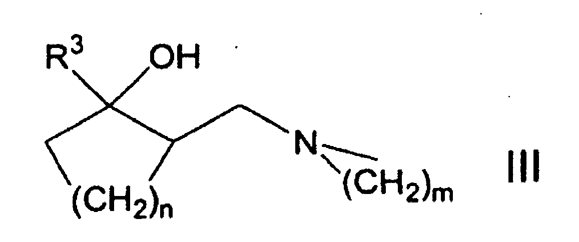 dans laquelle
n représente un nombre allant de 2 à 9 et m représente 6, dans les combinaisons suivantes
| n | m |
|---|---|
| 2 | 6 |
| 3 | 6 |
| 4 | 6 |
| 5 | 6 |
| 9 | 6 |
éventuellement un ou plusieurs groupes CH₂ de la chaîne (CH₂)ₘ pouvant être remplacés par un/des atomes d'oxygène.
R³ représente un radical aliphatique en C₁-C₃ linéaire ou ramifié, saturé ou insaturé, un radical cycloaliphatique en C₅-C₆ saturé ou insaturé, un radical phényle ou un radical hétéroaryle à cinq ou six chaînons, choisi dans l'ensemble comprenant les groupes pyrrolyle, furyle (furannyle), thiényle (thiophényle, pyrazolyle, imidazolyle, thiazolyle, oxazolyle, isoxazolyle, pyridinyle, pyridazinyle, pyrimidinyle, pyrazinyle et pyrannyle, le système cyclique respectif pouvant éventuellement être une ou plusieurs fois substitué par un atome d'halogène ou par un groupe alkyle, par un groupe alcoxy et/ou par un groupe alkyle trihalogéné et/ou être lié par un pont aliphatique en C₁-C₃ linéaire ou ramifié, saturé ou insaturé,
sous forme de leurs diastéréoisomères purs, de leurs énantiomères purs et de mélanges de ceux-ci - y compris de leurs racémates - ainsi que sous forme de bases correspondantes, de sels correspondants et de produits de solvatation correspondants.

11. Composés selon la revendication 10, **caractérisés en ce que**
R³ représente un radical vinyle, un radical cyclopentyle, un radical cyclohexyle, un radical thiophényle ou un radical phényle, le radical cyclohexyle pouvant éventuellement être lié par un pont méthylène ou le radical phényle pouvant éventuellement être une ou plusieurs fois substitué par un atome de fluor, de chlore, par un groupe méthyle, un groupe isopropyle, un groupe méthoxy et/ou un groupe trifluorométhyle et/ou éventuellement être lié par un pont aliphatique en C₁-C₃ linéaire, saturé, ou par un pont éthynyle.

12. Procédé pour la préparation de composés selon l'une quelconque des revendications 10-11, **caractérisé en ce que**
A) on fait réagir en chauffant une cétone de formule (1), dans laquelle n a la signification donnée dans la revendication 10, 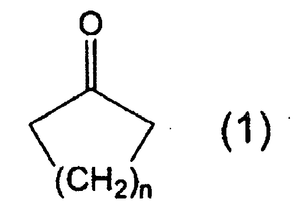 avec du paraformaldéhyde et une amine cyclique de formule (2), dans laquelle m a la signification donnée dans la revendication 10, 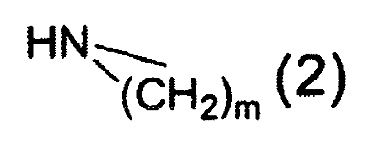 selon une réaction de Mannich, dans un solvant convenable, de préférence dans de l'éthanol, avec addition d'acide chlorhydrique ou dans de l'acide acétique, ensuite on soumet le mélange réactionnel à un traitement final, on isole le produit de formule (3) et éventuellement on le purifie, ou 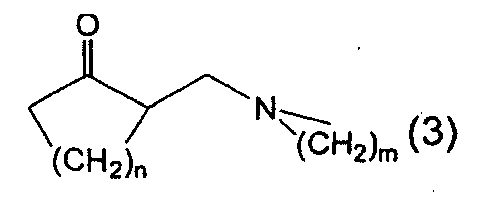
B) on fait réagir un composé de formule (3) avec un composé de Grignard ou un composé organolithié de formule R³MgCl, R³MgBr, R³MgI, MgR³₂ ou LiR³ où R³ a la signification donnée dans la revendication
10, dans un solvant convenable, de préférence l'oxyde d'éthyle ou le tétrahydrofuranne, ensuite on soumet le mélange réactionnel à un traitement final, on isole le composé de formule générale I et éventuellement on le purifie.
